(19) Europäisches Patentamt European Patent Office Office européen des brevets

(11) **EP 2 591 743 B1**

(12) **EUROPEAN PATENT SPECIFICATION**

(45) Date of publication and mention of the grant of the patent:
**11.07.2018 Bulletin 2018/28**

(51) Int Cl.:
***A61B 18/14*** *(2006.01)*

(21) Application number: **12191973.2**

(22) Date of filing: **09.11.2012**

(54) **Non-stick conductive coating for biomedical applications**

Nicht klebrige leitfähige Beschichtung für biomedizinische Anwendungen

Revêtement conducteur non collant pour applications biomédicales

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.11.2011 US 201113292575**

(43) Date of publication of application:
**15.05.2013 Bulletin 2013/20**

(73) Proprietor: **Colorado State University Research Foundation Fort Collins, CO 80522 (US)**

(72) Inventors:
• **Koo, Il-Gyo**
  **Fort Collins, CO 80526 (US)**
• **Kim, Paul Y.**
  **Fort Collins, CO 80521 (US)**
• **Kang, Sunggil**
  **790-784 Nam-Gu Pohang (KR)**
• **Collins, George J.**
  **Fort Collins, CO 80524 (US)**

(74) Representative: **Soames, Candida Jane et al Maschio & Soames IP Limited 30 Carlton Crescent Southampton SO15 2EW (GB)**

(56) References cited:
**WO-A1-00/16706          US-A- 5 201 900
US-A1- 2009 102 886**

## Description

## BACKGROUND

*Technical Field*

The present disclosure relates to medical devices, e.g., electrosurgical electrodes, having a non-stick, electrically conductive coating as well as methods and systems for applying the coating on the devices.

*Background of Related Art*

Currently, various medical devices include coatings, such as tracheal tubes, stents, implants, scalpels, instruments, fasteners, and the like. The coatings improve the quality of medical care provided using these devices. Examples of coatings include anti-clotting coatings, anti-bacterial coatings, anti-stick coatings, self-cleaning coatings, anticorrosion coatings, and the like. Various coatings have also been applied to electrosurgical electrodes used in energy-based tissue treatment.

Energy-based tissue treatment is well known in the art. Various types of energy (e.g., electrical, ultrasonic, microwave, cryogenic, heat, laser, etc.) are applied to tissue to achieve a desired result. Electrosurgery involves application of high radio frequency electrical current, microwave energy or resistive heating to a surgical site to cut, ablate, coagulate or seal tissue.

In bipolar electrosurgery, one of the electrodes of the hand-held instrument functions as the active electrode and the other as the return electrode. The return electrode is placed in close proximity to the active electrode such that an electrical circuit is formed between the two electrodes (e.g., electrosurgical forceps). In this manner, the applied electrical current is limited to the body tissue positioned between the electrodes.

Bipolar electrosurgical techniques and instruments can be used to coagulate blood vessels or tissue, e.g., soft tissue structures, such as lung, brain and intestine. A surgeon can either cauterize, coagulate, desiccate and/or simply reduce or slow bleeding, by controlling the intensity, frequency and duration of the electrosurgical energy applied between the electrodes and through the tissue. In order to achieve one of these desired surgical effects without causing unwanted charring of tissue at the surgical site or causing collateral damage to adjacent tissue, e.g., thermal spread, it is necessary to control the output from the electrosurgical generator, e.g., power, waveform, voltage, current, pulse rate, etc.

In monopolar electrosurgery, the active electrode is typically a part of the surgical instrument held by the surgeon that is applied to the tissue to be treated. A patient return electrode is placed remotely from the active electrode to carry the current back to the generator and safely disperse current applied by the active electrode. The return electrodes usually have a large patient contact surface area to minimize heating at that site. Heating is caused by high current densities which directly depend on the surface area. A larger surface contact area results in lower localized heat intensity. Return electrodes are typically sized based on assumptions of the maximum current utilized during a particular surgical procedure and the duty cycle (i.e., the percentage of time the generator is on).

The high temperatures involved in electrosurgery can cause charred matter to form and become affixed to the electrode tip. The buildup of charred matter can reduce the efficiency of the cutting and/or cauterizing processes by creating an insulating layer that interferes with the transference of RF energy to the targeted area. By way of example, when cauterizing an area to prevent bleeding, the charred matter can inhibit the cauterization, cause the destruction of additional tissue and increase thermal tissue damage. Thus, build-up of the charred matter can slow the surgical procedure, as the surgeon is required to remove the charred matter from the electrode tip.

The application of a fluoropolymer as a coating layer on at least a portion of an electrosurgical electrode tip has proven to be a valuable asset in providing additional properties to the tip, including providing a non-stick surface and high temperature stability. However, while the anti-adhesion properties of fluoropolymers, such as polytetrafluoroethylene ("PTFE"), as a coating layer on an electrode tip has facilitated electrosurgical cutting and/or cauterizing by reducing the build-up of debris on the electrode tip, it has not completely eliminated such build-up.

## SUMMARY

The present disclosure provides medical devices, e.g., electrosurgical electrodes, having a non-stick electrically conductive coating as well as systems and method for forming the coating. In embodiments, the coating may be a hydrophobic coating that is applied to the medical device under atmospheric conditions, e.g., atmospheric gases and pressure, using plasma enhanced chemical vapor deposition (AP-PECVD).

The present disclosure also provides for systems and methods for AP-PECVD used in open air to generate hydrophobic polymeric films. Plasmas have broad applicability to provide alternative solutions to industrial, scientific and medical needs, especially workpiece surface processing at low temperatures. Plasmas may be delivered to a workpiece, thereby affecting multiple changes in the properties of materials upon which the plasmas impinge. Plasmas have the unique ability to create large fluxes of radiation (e.g., ultraviolet), ions, photons, electrons and other excited-state (e.g., metastable) species which are suitable for modifying material properties with high spatial, material selectivity, and temporal control.

The present disclosure provides a plasma system including a plasma device having at least one electrode; an ionizable media source coupled to the plasma device and configured to supply ionizable media thereto; a precursor source configured to supply at least one monomer precursor to the plasma device; and a power source coupled to the at least one electrode and configured to ignite the ionizable media at the plasma device to form a plasma effluent at atmospheric conditions, wherein the plasma effluent polymerizes the at least one monomer precursor to form a hydrophobic, electrically-conductive polymer.

The present disclosure also provides a method for generating plasma. The method includes supplying ionizable media to a plasma device; igniting the ionizable media at the plasma device to form a plasma effluent at atmospheric conditions; contacting at least one monomer precursor with the plasma effluent; polymerizing the at least one monomer precursor to form a hydrophobic, electrically-conductive polymer; and depositing the hydrophobic, electrically conductive polymer on a surface of a workpiece to form a coating thereon.

The surface may form a tissue contacting surface of an electrosurgical electrode. A method of manufacturing an electrosurgical electrode may be provided, wherein the method includes the steps of the method above. A method of manufacturing an electrosurgical instrument may also be provided in which the workpiece forms an electrode thereof and the surface of the workpiece forms a tissue contacting surface of the electrode.

The method may include aerosolizing the monomer precursor. The system may include means configured to do so.

Electrodes for igniting the ionizable media, a downstream outlet of a first tube carrying the ionizable media, and a downstream outlet of a second tube carrying at least the monomer precursor may be arranged so that the monomer precursor is fed through the outlet of the second tube into the plasma effluent.

The second tube may be disposed radially inside the first tube. The first tube may extend so that the outlet of the first tube is axially beyond the outlet of the first tube. The first tube may form one of the electrodes. An ignition point for the ionizable medial may be disposed at a distal end of second tube so that the plasma effluent mixes with the monomer precursor as it exits from the second tube.

The method may comprise igniting the ionizable media in a state unmixed from the monomer precursor and mixing the monomer precursor directly into the ignited plasma effluent. The system may comprise means configured to do such.

The method may comprise flowing the monomer precursor into the plasma effluent so that the monomer precursor undergoes plasma induced polymerization, and the resulting polymers are carried by the plasma effluent to the surface of the workpiece. The system may comprise means for performing this process. Document US-A-2009/102886 discloses the most relevant prior art.

## BRIEF DESCRIPTION OF THE DRAWINGS

The accompanying drawings, which are incorporated in and constitute a part of this specification, illustrate exemplary embodiments of the disclosure and, together with a general description of the disclosure given above, and the detailed description of the embodiments given below, serve to explain the principles of the disclosure, wherein:

    Figs. 1A and B are perspective views of electrosurgical instruments according to an embodiment of the present disclosure;
Fig. 2 is a schematic diagram of a plasma system according to an embodiment of the present disclosure;
Fig. 3 is a perspective, cross-sectional perspective view of the plasma device according an embodiment to the present disclosure;
Figs. 4A and B are plots of contact angle as a function of input RF power and concentration of hexamethyldisiloxane of plasma-enhanced chemical vapor deposited hexamethyldisiloxane coatings; and
Figs. 5A and B are Fourier transform infrared (FTIR) spectrographs of plasma-enhanced chemical vapor deposited hexamethyldisiloxane coatings.

## DETAILED DESCRIPTION

The present disclosure provides for medical devices including, but not limited to, electrosurgical electrodes having a non-stick, electroconductive coating. Those skilled in the art will appreciate that the coating according to the present disclosure may be applied to other medical devices, such as tracheal tubes, wound covers, graspers, forceps, endoscopic tools, and the like.

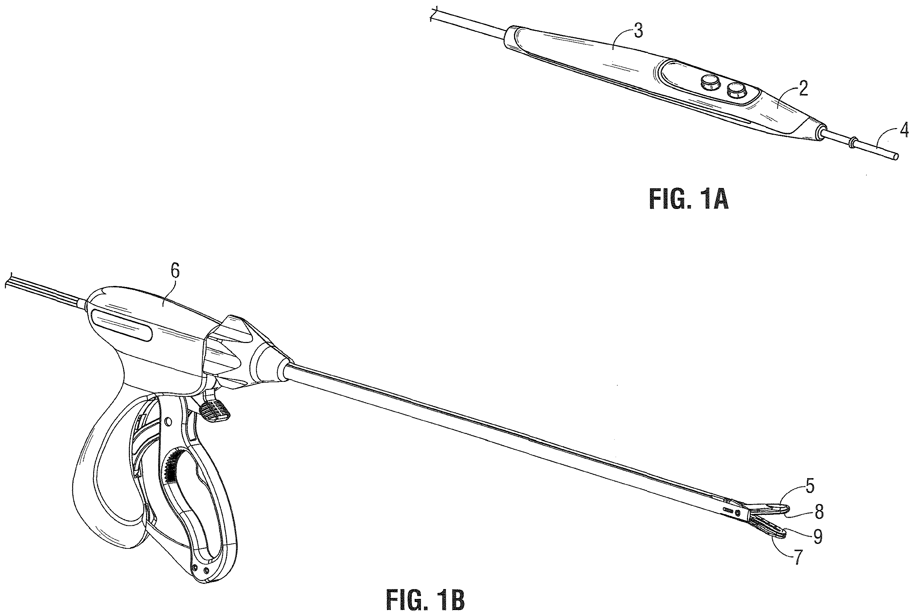

Fig. 1A shows a monopolar electrosurgical instrument 2 having a pencil-shaped housing 3. The electrosurgical instrument 2 includes an electrode 4 having a blade-like shape. In embodiments, the electrode 4 may have a variety of suitable shapes including, but not limited to, a loop, a hook, a paddle, a ball, and a roller. The electrode 4 may be removably coupled to the housing 3. The instrument 2 is configured to connect to an electrosurgical generator (not shown), which supplies electrosurgical energy for treating tissue (e.g., coagulate, cut, etc). A more detailed description of an electrosurgical pencil is found in a commonly-owned U.S. Patent No. 7,156, 842.

Fig. 1B shows a bipolar electrosurgical forceps 6 having one or more electrodes for treating tissue of a patient. In embodiments, the electrosurgical forceps 6 includes opposing jaw members 5 and 7 having one or more active electrodes 8 and a return electrode 9 disposed therein, respectively. The active electrode 8 and the return electrode 9 are connected to the electrosurgical generator which supplies electrosurgical energy to the forceps 6 for treating tissue grasped between the jaw members (e.g., sealing, coagulating, cutting, etc.).

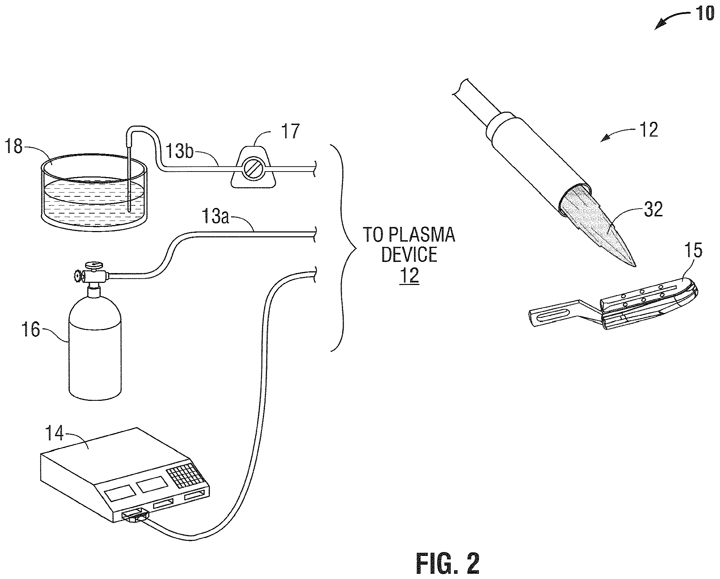

The electrodes 4, 8, 9 include a coating disposed on a surface thereof. In embodiments, the coating may be a hydrophobic, electrically conductive coating. The coating may include one or more hydrophobic, electrically conductive polymers formed from a monomer precursor. The coating may be deposited on the electrodes via a system 10 as shown in Fig. 2. The system 10 is configured to generate a plasma under atmospheric conditions. The term "atmospheric conditions" as used herein denotes an air-filled environment (e.g., an air gas mixture having oxygen, nitrogen, carbon dioxide, water, and other gases) at a temperature from about -10 °C to about 40 °C, in embodiments from about 0 °C to about 25 °C, and pressure from about 75 kPa to about 150 kPa, in embodiments from about 95 kPa to about 125 kPa.

The system 10 includes a plasma device 12 that is coupled to a power source 14, an ionizable media source 16 and a precursor or pre-ionization source 18. Power source 14 includes any suitable components for delivering power or matching impedance to plasma device 12. More particularly, the power source 14 may be any radio frequency generator or other suitable power source capable of producing electrical power to ignite and sustain the ionizable media to generate a plasma effluent 32.

Plasmas are generated using electrical energy that is delivered as either direct current (DC) electricity or alternating current (AC) electricity, in either continuous or pulsed modes, at frequencies from about 0.1 hertz (Hz) to about 100 gigahertz (GHz), including radio frequency bands ("RF", from about 0.1 MHz to about 100 MHz) and microwave bands ("MW", from about 0.1 GHz to about 100 GHz), using appropriate generators, electrodes, and antennas. AC electrical energy may be supplied at a frequency from about 0.1 MHz to about 2,450 MHz, in embodiments from about 1 MHz to about 160 MHz. The plasma may also be ignited by using continuous or pulsed direct current (DC) electrical energy or continuous or pulsed RF electrical energy or combinations thereof.

Choice of excitation frequency, the workpiece, as well as the electrical circuit that is used to deliver electrical energy to the circuit affects many properties and requirements of the plasma. The performance of the plasma chemical generation, the gas or liquid feedstock delivery system and the design of the electrical excitation circuitry are interrelated-- as the choices of operating voltage, frequency and current levels, as well as phase, effect the electron temperature and electron density. Further, choices of electrical excitation and plasma device hardware also determine how a given plasma system responds dynamically to the introduction of new ingredients to the host plasma gas or liquid media. The corresponding dynamic adjustment of the electrical drive, such as via dynamic match networks or adjustments to voltage, current, or excitation frequency may be used to maintain controlled power transfer from the electrical circuit to the plasma.

The precursor source 18 may include a bubbler or a nebulizer 17 configured to aerosolize precursor feedstocks prior to introduction thereof into the device 12. The nebulizer 17 may be one built by Analytica of Branford or may alternatively be a Burgener nebulizer (e.g., an Ari Mist model), in which the electrospray is used as an atomizer and is not energized electrically. In embodiments, the precursor source 18 may also include a micro droplet or injector system capable of generating predetermined refined droplet volume of the precursor feedstock from about 1 femtoliter to about 1 nanoliter in volume. The precursor source 18 may also include a microfluidic device, a piezoelectric pump, or an ultrasonic vaporizer.

The system 10 provides a flow of plasma through the device 12 to a workpiece 15 (e.g., electrodes 4, 8, 9 to be coated). Plasma feedstocks, which include ionizable media and precursor feedstocks, are supplied by the ionizable media source 16 and the precursor source 18, respectively, to the plasma device 12. During operation, the precursor feedstock and the ionizable media are provided to the plasma device 12 where the plasma feedstocks are ignited to form plasma effluent 32. The feedstocks may be mixed upstream from the ignition point or midstream thereof (e.g., at the ignition point) of the plasma effluent, as shown in Fig. 1 and described in more detail below.

The ionizable media source 16 provides ionizable feedstock gas mix to the plasma device 12. The ionizable media source 16 is coupled to the plasma device 12 and may include a storage tank and a pump (not explicitly shown). The ionizable media may be a liquid or a gas such as argon, helium, neon, krypton, xenon, radon, carbon dioxide, nitrogen, hydrogen, oxygen, etc. and their mixtures, and the like. These and other gases may be initially in a liquid form that is gasified during application.

The precursor source 18 provides precursor feedstock to the plasma device 12. The precursor feedstock may be either in solid, gaseous or liquid form and may be mixed with the ionizable media in any state, such as solid, liquid (e.g., particulates, nanoparticles or droplets), gas, and the combination thereof. The precursor source 18 may include a heater, such that if the precursor feedstock is liquid, it may be heated into gaseous state prior to mixing with the ionizable media.

In one embodiment, the precursors may be any chemical species capable of forming a hydrophobic, electrically conductive coating on the workpiece. In embodiments, the precursor may be any monomer that may be polymerized by the plasma. Examples of suitable monomers include, but are not limited to, alkyl acrylates such as n-butyl acrylate, tertbutyl acrylate, 2-ethylhexyl acrylate, lauryl acrylate, and the like; alkanes, such as methane, ethane, butane, and the like; alkynes, such as styrene, acetylene, and the like; fluorocarbons such as carbon tetrafluoride, octafluorocyclobutane, hexafluoroacetone, tetrafluoroethane, hexafluoropropylene, perfluorobutane, and other fluorocarbons having a fluoride to carbon ratio of less than 3; organosilicones such as silane, hexamethyldisiloxane (HMDSO), and the like, as well as mixtures, such as carbon tetrafluoride, butane, and acetylene, carbon tetrafluoride and methane, octafluorocyclobutane and methane, and combinations thereof.

The precursor materials are mixed with the ionizable media and are volatized and/or polymerized and are then deposited on the workpiece 15 by the plasma effluent 32. In particular, the precursors react with the reactive species of the plasma effluent 32, such as ions, electrons, excited-state (e.g., metastable) species, molecular fragments (e.g., radicals) and the like, which are formed when the ionizable media is ignited by electrical energy from the power source 14.

The ionizable media flow rate may be from about 500 standard cubic centimeters per minute (SCCM) to about 1,200 SCCM, in embodiments from about 800 SCCM to about 900 SCCM. The concentration of the monomer precursor to the ionizable media may be from about 0.1% to about 5% by volume of the ionizable media, in embodiments from about 0.25% to about 2% by volume of the ionizable media in further embodiments from about 0.5% to about 1% by volume of the ionizable media.

The ionizable media source 16 and the precursor source 18 and may be coupled to the plasma device 12 via tubing 13a and 13b, respectively. The tubing 13a and 13b may be combined into a single tubing (e.g., via a Y coupling) to deliver a mixture of the ionizable media and the precursor feedstock to the device 12 at a proximal end thereof. This allows for the plasma feedstocks, e.g., the precursor feedstocks, nanoparticles and the ionizable gas, to be delivered to the plasma device 12 simultaneously prior to ignition of the mixture therein.

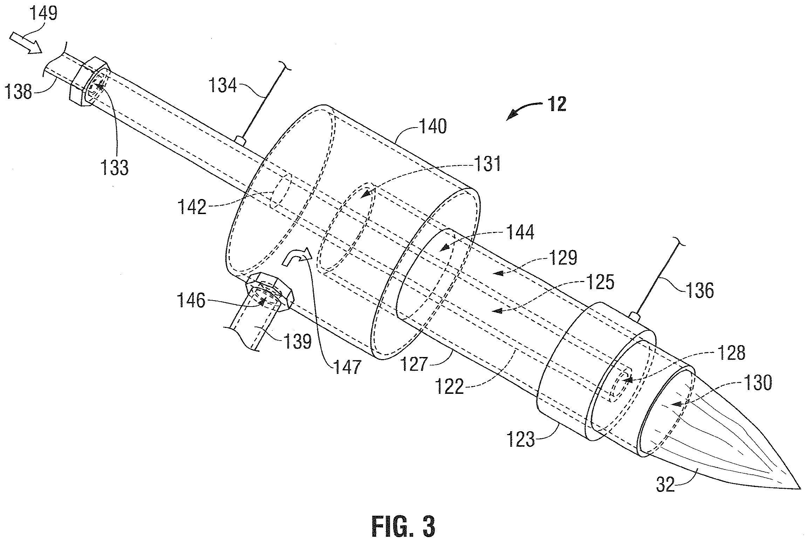

In another embodiment, the ionizable media source 16 and the precursors source 18 may be coupled to the plasma device 12 via the tubing 13a and 13b at separate connections as shown in Fig. 3, such that the mixing of the feedstocks occurs within the plasma device 12 upstream from the ignition point. In other words, the plasma feedstocks are mixed proximally of the ignition point, which may be any point between the respective sources 16 and 18 and the plasma device 12, prior to ignition of the plasma feedstocks to create the desired mix of the plasma effluent species flux (e.g., particles/cm$^2$sec) for each specific surface treatment on the workpiece "W."

In a further embodiment, the plasma feedstocks may be mixed midstream, e.g., at the ignition point or downstream of the plasma effluent, directly into the plasma effluent 32. More specifically, the tubing 13a and 13b may be coupled to the device 12 at the ignition point, such that the precursor feedstocks and the ionizable media are ignited concurrently as they are mixed. It is also envisioned that the ionizable media may be supplied to the device 12 proximally of the ignition point, while the precursor feedstocks are mixed therewith at the ignition point.

In a further illustrative embodiment, the ionizable media may be ignited in an unmixed state and the precursors may be mixed directly into the ignited plasma effluent 32. Prior to mixing, the plasma feedstocks may be ignited individually. The plasma feedstock is supplied at a predetermined pressure to create a flow of the medium through the device 12, which aids in the reaction of the plasma feedstocks and produces the plasma effluent 32. The plasma effluent 32 according to the present disclosure is generated at or near atmospheric pressure under normal atmospheric conditions.

With reference to Fig. 3, the device 12 includes an inner electrode 122 disposed coaxially within a first housing 127. The inner electrode 122 has a substantially cylindrical tubular shape defining a lumen 125 therein. The inner electrode 122 includes a proximal opening 133 and a distal opening 128. The inner electrode 122 is coupled to the precursor source 18 via the tubing 13b at the distal opening 128. The first housing 127 also has a substantially cylindrical tubular shape defining a lumen 129 therethrough with the inner electrode 122 disposed therein. In particular, the first housing 127 includes a distal opening 130 and a proximal opening 131.

The device 12 also includes an outer electrode 123. The outer electrode 123 also has a substantially cylindrical tubular shape and is disposed over the outer surface of the first housing 127. The electrodes 122 and 123 may be formed from a conductive material suitable for ignition of plasma such as metals and metal-ceramic composites. In one embodiment, the electrodes 122 and 123 may be formed from a conductive metal including a native oxide or nitride compound disposed thereon. In embodiments, the first housing 127 may be formed from a dielectric material, such as ceramic, plastic, and the like, to provide for capacitive coupling between the inner and outer electrodes 122 and 123.

The proximal portion of the first housing 127, namely the opening 131, is disposed within a second housing 140. The second housing 140 includes a proximal opening 142 and a distal opening 144. The inner electrode 122 is inserted through the proximal opening 142 and is coupled to the second housing 140 at that junction. The first housing 127 is inserted through the distal opening 144 and is also coupled to the second housing 140 at that junction. The second housing 140 also includes an inlet 146 coupled to the ionizable media source 16 via the tubing 13a.

One of the electrodes 122 and 123 may be an active electrode and the other may be a neutral (e.g., indifferent) or return electrode to facilitate RF energy coupling through an isolation transformer (not shown) disposed within the generator 14 to provide electrical isolation with the workpiece "W." Each of the electrodes 122 and 123 is coupled to the power source 14 via leads 134 and 136, respectively. The power source 14 drives plasma generation such that the energy from the power source 14 may be used to ignite and the plasma feedstocks flowing through the device 12. Applied power to the electrodes 122 and 123 for generation of the plasma effluent 32 may be from about 10 watts (W) to about 50 W, in embodiments from about 20 W to about 30 W.

The ionizable media and the precursors flow through the device 12 through the inlet 146 and the opening 133 as shown by arrows 147 and 149, respectively. The plasma effluent 32 is generated within the lumen 129 as the ionizable media passes between the inner and outer electrodes 122 and 123, which are capacitively coupled through the first housing 127. The monomer precursors are fed through the lumen 125 of the inner electrode 122 directly into the plasma effluent 32. Upon flowing into the plasma effluent 32, the monomer precursors undergo plasma-induced polymerization. In particular, the highly reactive radicals including, but not limited to, hydroxyl, oxygen, hydrogen radicals, induce a variety of polymerization reactions with the monomer precursors. The resulting polymers are carried by the plasma effluent 32 to the surface of the workpiece 15 as shown in Fig. 2. The resulting hydrophobic, electrically-conductive coating may have a hydrophobicity expressed by a contact angle at which the liquid (e.g., water) contacts the surface of the workpiece 15. The contact angle may be from about 80° to about 120°, in embodiments from about 90° to about 115°.

The inner electrode 122 may include a coating formed from an insulative or semiconductive material deposited as a film (e.g., atomic layer deposition) or as a dielectric sleeve or layer. In embodiments, the coating may be disposed on the outer and inner surface of the inner electrode 122. In one embodiment, the coating may cover the entire surface of the inner electrode 122 (e.g., outer and inner surface thereof, respectively). In another embodiment, the coating may cover only a portion of the inner electrode 122.

The coating may be a nanoporous native oxide, or a native nitride of the metal from which the inner and outer electrodes are formed, or may be a deposited layer or a layer formed by ion implantation. In embodiments, the inner electrode 122 is formed from an aluminum alloy and the coating is aluminum oxide ($Al_2O_3$) or aluminum nitride (AlN). In another illustrative embodiment, the inner electrode 122 is formed from a titanium alloy and the coating is titanium oxide ($TiO_2$) or titanium nitride (TiN). In embodiments, the coating may also be a non-native metal oxide or nitride, such as zinc oxide ($ZnO_2$) and magnesium oxide (MgO). The coating may also be used to reduce tissue sticking to the electrode.

The inner electrode 122 and the coating may also be configured as a heterogeneous system, in which the inner electrode 122 is formed from one material and the coating is formed from another material. In particular, the inner electrode 122 may be formed from any suitable electrode substrate material (e.g., conductive metal or a semiconductor) and the coating may be disposed thereon by various coating processes. The coating may be formed on the inner electrode 122 by exposure to an oxidizing environment, anodization, electrochemical processing, ion implantation, or deposition (e.g., sputtering, chemical vapor deposition, atomic layer deposition, etc.).

In embodiments, the coating provides for capacitive coupling between the inner electrode 122 and the outer electrode 123 in addition to the first housing 127. The resulting capacitive circuit element structure provides for a net negative bias potential at the surface of the inner electrode 122, which attracts the ions and other species from the plasma effluent. These species then bombard the coating and release energetic electrons.

Materials having high secondary electron emission property, γ, in response to ion and/or photon bombardment are suitable for forming the coating. Such materials include insulators and/or semiconductors. These materials have a relatively high γ, where γ represents the number of electrons emitted per incident bombardment particle. Thus, metals generally have a low γ (e.g., less than 0.1) while insulative and semiconductor materials, such as metallic oxides have a high γ, from about 1 to about 10 with some insulators exceeding a value of 20. Thus, the coating acts as a source of secondary emitted electrons.

Secondary electron emission, γ, may be described by the formula (1):

$$(1) \qquad \gamma = \Gamma_{secondary}/\Gamma_{ion}$$

In formula (1) γ is the secondary electron emission yield or coefficient, $\Gamma_{secondary}$ is the electron flux, and $\Gamma_{ion}$ is the ion flux. Secondary emission occurs due to the impacts of plasma species (e.g., ions) onto the coating when the ion impact collisions have sufficient energy to induce secondary electron emission, thus generating γ-mode discharges. Generally discharges are said to be in γ-mode when electron generation occurs at electrode surfaces (i.e., γ > 1) instead of in the gas (an α-mode discharge). In other words, per each ion colliding with the coating, a predetermined number of secondary electrons are emitted. Thus, γ may also be thought of as a ratio of the $\Gamma_{secondary}$ (e.g., the electron flux) and $\Gamma_{ion}$ (e.g., the ion flux).

These ion collisions with the surface of the coating, in turn, provide sufficient energy for secondary electron emission to generate γ discharges. The ability of coating materials to generate γ discharges varies with several parameters, with the most influence due to the choice of materials having a high γ as discussed above. This property allows coating to act as a source of secondary emitted electrons or as a catalytic material to enhance selected chemical reaction paths.

Over time the coating may thin or be removed during the plasma operation. In order to maintain the coating to continually provide a source of secondary emitted electrons, the coating may be continually replenished during the plasma operation. This may be accomplished by adding species that reformulate the native coating on the inner electrode 122. In one embodiment, the precursor source 18 may provide either oxygen or nitrogen gas to the device 12 to replenish the oxide or nitride coating.

Conventional non-atmospheric PECVD require an expensive low pressure vacuum chamber and load-locked batch processing. The AP-PECVD according to the present disclosure present a significant advantage over non-atmospheric PECVD in that the processing can occur in open air as part of in-line manufacturing. Moreover, AP-PECVD is also performed at a relatively low temperature so that temperature-sensitive substrates may be coated without thermal damage. Further, conventional chemical polymerization processes require a relatively long reaction time and/or use of catalysts to reduce reaction time. In the AP-PECVD according to the present disclosure plasma-generated reactive radicals break the chemical bonds of monomers precursors to generate and form an unstable intermediate molecule, which when deposited on the surface of the workpiece polymerizes to form a stable polymeric film.

The following Examples are being submitted to illustrate embodiments of the present disclosure. These Examples are intended to be illustrative only and are not intended to limit the scope of the present disclosure. Also, parts and percentages are by weight unless otherwise indicated. As used herein, "room temperature" refers to a temperature of from about 20 °C to about 30° C.

Example 1 - Hydrophobic Coating by Argon Plasma Polymerization of Hexamethyldisiloxane (HMDSO).

A plasma system was setup according to Figs. 2 and 3 and argon gas was supplied to the electrodes at a flow rate from about 800 cubic centimeters per minute (SCCM) to about 900 SCCM. Radio frequency (RF) power was supplied to the electrodes at about 25 watts (W). HMDSO was then supplied to the plasma at a concentration from about 0.2% to about 2.0% by volume of the argon gas. The plasma effluent was applied to a glass substrate. Concentration of HMDSO and RF power were varied to obtain multiple coated substrates.

Example 2 - Hydrophobicity of the Coated Substrates.

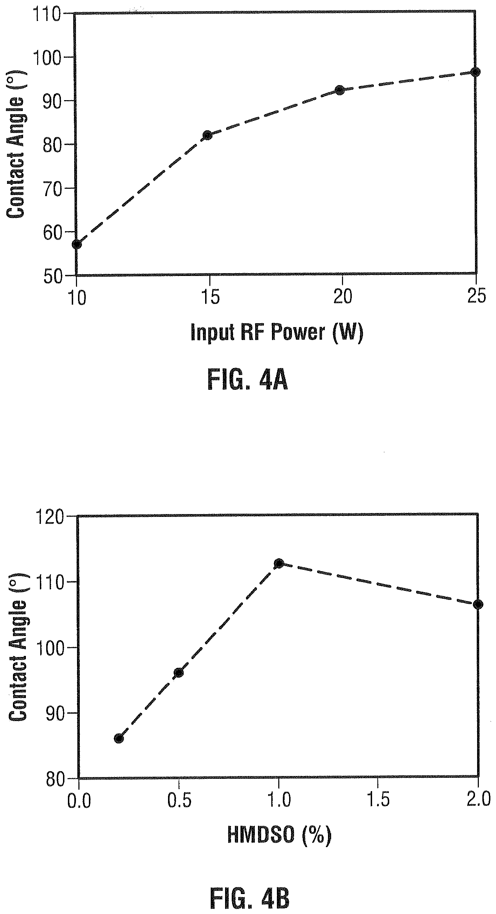

Hydrophobicity of the coatings was analyzed by measuring the contact angle of water droplets on the surface of the coated substrates. Figs. 4A and 4B are graphs of the contact angle as a function of input RF power and concentration of HMDSO, respectively. In particular, Fig. 4A shows that the contact angle increased, e.g., the coating was more hydrophobic, as the RF power was increased. Argon flow rate was maintained at about 800 sccm and concentration of HMDSO was about 0.5%. Fig. 4B shows that the largest contact angle occurred at the concentration of the HMDSO being about 0.5%. Argon flow rate was also maintained at about 800 sccm and RF power was about 25 W.

Example 3 - Chemical Structure of the Coatings.

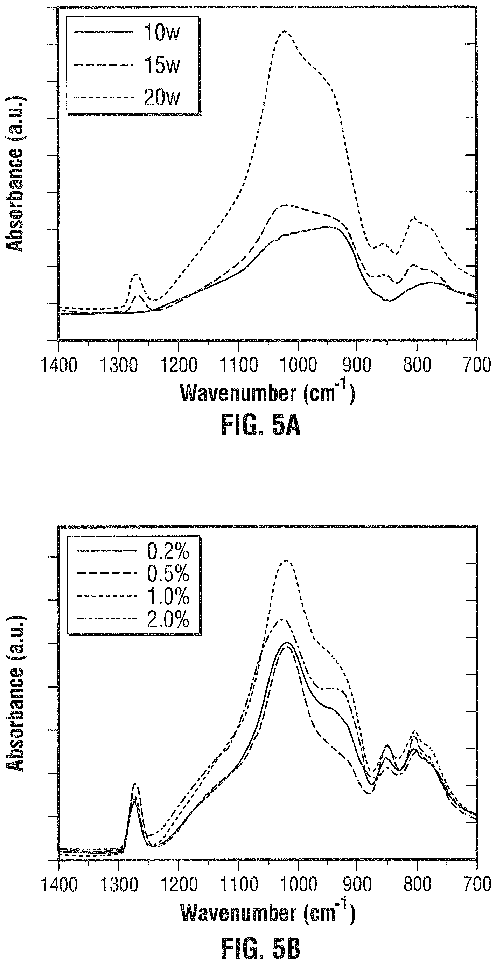

Coatings deposited under input RF power from about 10 W to about 20 W and HMDSO concentration from about 0.2% to about 2% were analyzed via Fourier transform infrared (FTIR) spectroscopy. Figs. 5A and 5B show FTIR spectra of the coatings. Fig. 5A shows spectra of the glass substrates coated with three (3) HMDSO polymeric coatings deposited by plasma generated at RF power of about 10 W, 15 W, and 20 W with the argon flow rate being about 800 sccm and HMDSO concentration of about 1%. Fig. 5B shows spectra of the glass substrates coated with four (4) HMDSO polymeric coatings deposited by plasma generated at RF power of about 25 W with the argon flow rate being about 800 sccm and HMDSO concentration of about 0.2%, 0.5%, 1%, and 2%.

Although the illustrative embodiments of the present disclosure have been described herein with reference to the accompanying drawings, it is to be understood that the disclosure is not limited to those precise embodiments, and that various other changes and modifications may be effected therein by one skilled in the art without departing from the scope of the disclosure. In particular, as discussed above this allows the tailoring of the relative populations of plasma species to meet needs for the specific process desired on the workpiece surface or in the effluent of the reactive plasma.

**Claims**

1. A plasma system (10) comprising:

   a plasma device (12) including;

      a first housing (127) defining a first lumen (129), the first housing including a proximal opening and a distal opening;
      a second housing (140) being in gaseous communication with the first lumen;
      an outer electrode (123) having a substantially cylindrical tubular shape, the outer electrode is disposed over the first housing; and
      an inner electrode (122) having a substantially cylindrical tubular shape and defining a second lumen (125) therethrough, the inner electrode coaxially disposed within the first lumen and extending proximally past a proximal end of the second housing, the inner electrode including a proximal opening and a distal opening; the proximal opening of the first housing being disposed within the second housing, the second housing including a proximal opening and a distal opening, the inner electrode being inserted through the proximal opening of the second housing and being coupled to the second housing at that junction, the first housing being inserted through the distal opening of the second housing and being also coupled to the second housing at said junction, the second housing including an inlet; an ionizable media source (16) coupled to the inlet of the second housing and configured to supply ionizable media thereto;

a precursor source (18) coupled to the proximal opening of the inner electrode and configured to supply at least one monomer precursor thereto; and
a power source (14) coupled to the inner and outer electrodes and configured to ignite the ionizable media at a distal opening (130) of the first housing to form a plasma effluent at atmospheric conditions, wherein the second lumen defined through the inner electrode supplies the at least one monomer precursor into the plasma effluent such that the plasma effluent polymerizes the at least one monomer precursor to form a hydrophobic, electrically-conductive polymer.

2. The plasma system according to claim 1, wherein at least one of the inner electrode or the outer electrode is formed from a metal alloy selected from the group consisting of an aluminum alloy and a titanium alloy.

3. The plasma system according to claim 1 or 2, wherein the at least one monomer precursor is selected from the group consisting of n-butyl acrylate, tertbutyl acrylate, 2-ethylhexyl acrylate, lauryl acrylate, methane, ethane, butane, styrene, acetylene, carbon tetrafluoride, octafluorocyclobutane, hexafluoroacetone, tetrafluoroethane, hexafluoropropylene, perfluorobutane, silane, hexamethyldisiloxane, and combinations thereof.

4. The plasma system according to claim 1, 2 or 3, wherein the precursor source includes a nebulizer configured to form an aerosol spray of the at least one monomer precursor.

5. A method for generating plasma by using the plasma system of any of the preceding claims comprising:

supplying ionizable media to the plasma device;
igniting the ionizable media at the plasma device to form a plasma effluent at atmospheric conditions;
contacting at least one monomer precursor with the plasma effluent, wherein the at least one monomer precursor includes at least one catalyst material;

polymerizing the at least one monomer precursor to form a hydrophobic, electrically-conductive polymer; and
depositing the hydrophobic, electrically conductive polymer on a surface of a workpiece to form a coating thereon.

6. The method according to claim 5, wherein the ionizable media is supplied at a flow rate from about 800 sccm to about 900 sccm.

7. The method according to claim 5 or 6, wherein the at least one monomer precursor is supplied at a concentration from about 0.25% to about 2% by volume of the ionizable media.

8. The method according to any one of claims 5 to 7, wherein the igniting further comprises supplying radio frequency power to the ionizable media from about 10 watts to about 50 watts.

9. The method according to any one of claims 5 to 8, wherein the at least one monomer precursor is selected from the group consisting of n-butyl acrylate, tertbutyl acrylate, 2-ethylhexyl acrylate, lauryl acrylate, methane, ethane, butane, styrene, acetylene, carbon tetrafluoride, octafluorocyclobutane, hexafluoroacetone, tetrafluoroethane, hexafluoropropylene, perfluorobutane, silane, hexamethyldisiloxane, and combinations thereof.

10. The method according to any one of claims 5 to 9, wherein the coating has a hydrophobicity expressed by a contact angle from about 80° to about 120°.

**Patentansprüche**

1. Plasmasystem (10), umfassend:

eine Plasmavorrichtung (12), aufweisend:

ein erstes Gehäuse (127), das ein erstes Lumen (129) definiert;
wobei das erste Gehäuse eine proximale Öffnung und eine distale Öffnung aufweist;
ein zweites Gehäuse (140), das in gasförmiger Verbindung mit dem ersten Lumen steht;
eine äußere Elektrode (123), die eine im Wesentlichen zylindrische Röhrenform aufweist, wobei die äußere Elektrode über dem ersten Gehäuse angeordnet ist; und
eine innere Elektrode (122), die eine im Wesentlichen zylindrische Röhrenform aufweist und ein zweites Lumen (125) dadurch definiert, wobei die innere Elektrode koaxial innerhalb des ersten Lumens angeordnet ist und sich proximal über ein proximales Ende des zweiten Gehäuses hinaus erstreckt, wobei die innere Elektrode eine proximale Öffnung und eine distale Öffnung aufweist;

wobei die proximale Öffnung des ersten Gehäuses innerhalb des zweiten Gehäuses angeordnet ist, wobei das zweite Gehäuse eine proximale Öffnung und eine distale Öffnung aufweist, wobei die innere Elektrode durch die proximale Öffnung des zweiten Gehäuses eingeführt wird und an diesem Verbindungsstück mit dem zweiten Gehäuse gekoppelt ist, wobei das erste Gehäuse durch die distale Öffnung des zweiten Gehäuses eingeführt ist und auch an dem Verbindungsstück mit dem zweiten Gehäuse gekoppelt ist, wobei das zweite Gehäuse einen Einlass aufweist;

eine ionisierbare Medienquelle (16), die mit dem Einlass des zweiten Gehäuses gekoppelt ist und konfiguriert ist, um ionisierbare Medien zuzuführen;

eine Vorläuferquelle (18), die mit der proximalen Öffnung der inneren Elektrode gekoppelt ist und konfiguriert ist um, mindestens ein Monomervorläufer zuzuführen; und

eine Stromquelle (14), die mit der inneren und äußeren Elektrode gekoppelt ist und konfiguriert ist, um die ionisierbaren Medien an einer distalen Öffnung (130) des ersten Gehäuses zu zünden, um bei atmosphärischen Bedingungen einen Plasmaausfluss zu bilden, wobei das zweite Lumen, das durch die innere Elektrode definiert ist, den mindestens einen Monomervorläufer dem Plasmaausfluss zuführt, sodass der Plasmaausfluss den mindestens einen Monomervorläufer polymerisiert, um ein hydrophobes, elektrisch leitfähiges Polymer zu bilden.

2. Plasmasystem nach Anspruch 1, wobei mindestens eine von der inneren Elektrode oder der äußeren Elektrode aus einer Metalllegierung gebildet ist, die ausgewählt ist aus der Gruppe bestehend aus einer Aluminiumlegierung und einer Titanlegierung.

3. Plasmasystem nach Anspruch 1 oder 2, wobei der mindestens eine Monomervorläufer ausgewählt ist aus der Gruppe bestehend aus n-Butylacrylat, tert-Butylacrylat, 2-Ethylhexylacrylat, Laurylacrylat, Methan, Ethan, Butan, Styrol, Acetylen, Tetrafluorkohlenstoff, Octafluorcyclobutan, Hexafluoraceton, Tetrafluorethan, Hexafluorpropylen, Perfluorbutan, Silan, Hexamethyldisiloxan und Kombinationen davon.

4. Plasmasystem nach Anspruch 1, 2 oder 3, wobei die Vorläuferquelle einen Zerstäuber umfasst, der konfiguriert ist, um ein Aerosolspray des mindestens einen Monomervorläufers zu bilden.

5. Verfahren zur Erzeugung von Plasma unter Verwendung des Plasmasystems nach einem der vorstehenden Ansprüche, umfassend:

Zuführen ionisierbarer Medien zu der Plasmavorrichtung;

Zünden der ionisierbaren Medien an der Plasmavorrichtung, um einen Plasmaausfluss bei atmosphärischen Bedingungen zu bilden;

Inkontaktbringen von mindestens einem Monomervorläufer mit dem Plasmaausfluss, wobei der mindestens eine Monomervorläufer mindestens ein Katalysatormaterial aufweist;

Polymerisieren des mindestens einen Monomervorläufers, um ein hydrophobes, elektrisch leitfähiges Polymer zu bilden; und

Abscheiden des hydrophoben, elektrisch leitfähigen Polymers auf eine Oberfläche eines Werkstücks zum Bilden einer Beschichtung darauf.

6. Verfahren nach Anspruch 5, wobei die ionisierbaren Medien mit einer Durchflussrate von etwa 800 sccm bis etwa 900 sccm zugeführt werden.

7. Verfahren nach Anspruch 5 oder 6, wobei der mindestens eine Monomervorläufer bei einer Konzentration von etwa 0,25 Vol.-% bis etwa 2 Vol.-% der ionisierbaren Medien zugeführt wird.

8. Verfahren nach einem der Ansprüche 5 bis 7, wobei das Zünden ferner das Zuführen von Hochfrequenzenergie von etwa 10 Watt bis etwa 50 Watt zu den ionisierbaren Medien umfasst.

9. Verfahren nach einem der Ansprüche 5 bis 8, wobei der mindestens eine Monomervorläufer ausgewählt wird aus der Gruppe bestehend aus n-Butylacrylat, tert-Butylacrylat, 2-Ethylhexylacrylat, Laurylacrylat, Methan, Ethan, Butan, Styrol, Acetylen, Tetrafluorkohlenstoff, Octafluorcyclobutan, Hexafluoraceton, Tetrafluorethan, Hexafluorpropylen, Perfluorbutan, Silan, Hexamethyldisiloxan und Kombinationen davon.

10. Verfahren nach einem der Ansprüche 5 bis 9, wobei die Beschichtung eine Hydrophobie aufweist, die ausgedrückt wird durch einen Kontaktwinkel von etwa 80° bis etwa 120°.

**Revendications**

1. Système à plasma (10) comprenant :

un dispositif à plasma (12) incluant :

un premier logement (127) définissant une première lumière (129), le premier logement incluant une ouverture proximale et une ouverture distale ;

un second logement (140) étant en communication gazeuse avec la première lumière ;

une électrode externe (123) ayant une forme tubulaire sensiblement cylindrique, l'électrode externe étant disposée au-dessus du premier logement ; et

une électrode interne (122) ayant une forme tubulaire sensiblement cylindrique et définissant une seconde lumière (125) à travers elle, l'électrode interne étant disposée coaxialement à l'intérieur de la première lumière et s'étendant proximalement au-delà d'une extrémité proximale du second logement, l'électrode interne incluant une ouverture proximale et une ouverture distale ;

l'ouverture proximale du premier logement étant disposée à l'intérieur du second logement, le second logement incluant une ouverture proximale et une ouverture distale, l'électrode interne étant insérée à travers l'ouverture proximale du second logement et étant couplée au second logement au niveau de cette jonction, le premier logement étant inséré à travers l'ouverture distale du second logement et étant également couplé au second logement au niveau de ladite jonction, le second logement incluant une entrée ;

une source de milieu ionisable (16) couplée à l'entrée du second logement et configurée pour lui fournir un milieu ionisable ;

une source de précurseur (18) couplée à l'ouverture proximale de l'électrode interne et configurée pour lui fournir au moins un précurseur monomère ; et

une source d'alimentation (14) couplée aux électrodes interne et externe et configurée pour allumer le milieu ionisable au niveau d'une ouverture distale (130) du premier logement pour former un effluent de plasma à des conditions atmosphériques, dans lequel la seconde lumière définie à travers l'électrode interne fournit l'au moins un précurseur monomère dans l'effluent de plasma de telle sorte que l'effluent de plasma polymérise l'au moins un précurseur monomère pour former un polymère électroconducteur hydrophobe.

2.  Système à plasma selon la revendication 1, dans lequel au moins l'une de l'électrode interne ou de l'électrode externe est formée à partir d'un alliage de métal choisi dans le groupe constitué d'un alliage d'aluminium et d'un alliage de titane.

3.  Système à plasma selon la revendication 1 ou 2, dans lequel l'au moins un précurseur monomère est sélectionné dans le groupe constitué d'acrylate de n-butyle, acrylate de terbutyle, acrylate de 2-éthylhexyle, acrylate de lauryle, méthane, éthane, butane, styrène, acétylène, tétrafluorure de carbone, octafluorocyclobutane, hexafluoroacétone, tétrafluoroéthane, hexafluoropropylène, perfluorobutane, silane, hexaméthyldisiloxane et de combinaisons de ceux-ci.

4.  Système à plasma selon la revendication 1, 2 ou 3, dans lequel la source de précurseur inclut un nébuliseur configuré pour former une pulvérisation aérosol de l'au moins un précurseur monomère.

5.  Procédé pour générer un plasma au moyen du système à plasma selon l'une quelconque des revendications précédentes comprenant :

la fourniture de milieu ionisable au dispositif à plasma ;

l'allumage du milieu ionisable au niveau du dispositif à plasma pour former un effluent de plasma à des conditions atmosphériques ;

la mise en contact d'au moins un précurseur monomère avec l'effluent de plasma, dans lequel l'au moins un précurseur monomère inclut au moins un matériau catalyseur ;

la polymérisation de l'au moins un précurseur monomère pour former un polymère électroconducteur hydrophobe ; et

le dépôt du polymère électroconducteur hydrophobe sur une surface d'une pièce à travailler pour former un revêtement sur celle-ci.

6.  Procédé selon la revendication 5, dans lequel le milieu ionisable est fourni à un débit d'environ 800 $cm^3$/min standard à environ 900 $cm^3$/min standard.

7.  Procédé selon la revendication 5 ou 6, dans lequel l'au moins un précurseur monomère est fourni à une concentration d'environ 0,25 % à environ 2 % en volume du milieu ionisable.

8.  Procédé selon l'une quelconque des revendications 5 à 7, dans lequel l'allumage comprend en outre la fourniture d'une puissance radiofréquence au milieu ionisable d'environ 10 watts à environ 50 watts.

9.  Procédé selon l'une quelconque des revendications 5 à 8, dans lequel l'au moins un précurseur monomère est sélectionné dans le groupe constitué d'acrylate de n-butyle, acrylate de terbutyle, acrylate de 2-éthylhexyle, acrylate de lauryle, méthane, éthane, butane, styrène, acétylène, tétrafluorure de carbone, octafluorocyclobutane, hexafluoroacétone, tétrafluoroéthane, hexafluoropropylène, perfluorobutane, silane, hexaméthyldisiloxane et de combinaisons de ceux-ci.

10. Procédé selon l'une quelconque des revendications 5 à 9, dans lequel le revêtement possède un caractère hydrophobe exprimé par un angle de contact d'environ 80° à environ 120°.

FIG. 1A

FIG. 1B

**FIG. 2**

EP 2 591 743 B1

**FIG. 3**

EP 2 591 743 B1

**FIG. 4A**

**FIG. 4B**

FIG. 5A

FIG. 5B

16

**EP 2 591 743 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2009102886 A
- US 7156842 B